# EUROPÄISCHE PATENTSCHRIFT

(11) **EP 1 859 830 B1**
(45) Veröffentlichungstag und Bekanntmachung des Hinweises auf die Patenterteilung: **03.01.2018**
(21) Anmeldenummer: 07006673.3
(22) Anmeldetag: 30.03.2007
(51) Int. Cl.: A61M 16/00, A61N 1/39, A61H 31/00

(54) **Vorrichtung zur Notfallversorgung**
Emergency supply device
Dispositif destiné à l'alimentation d'urgence

(30) Priorität: 01.04.2006 DE 102006016124
(43) Veröffentlichungstag der Anmeldung: 28.11.2007
(73) Patentinhaber: Weinmann Emergency Medical Technology GmbH + Co. KG, 22525 Hamburg (DE)
(72) Erfinder: Palm, Ulrich, 22848 Norderstedt (DE); Szepannek, Martin, 25436 Uetersen (DE)
(74) Vertreter: Patentanwälte Klickow & Partner Partnerschaftsgesellschaft mbB

(56) Entgegenhaltungen:
- EP-A- 0 903 141
- EP-A- 1 264 615
- EP-A- 1 702 649
- WO-A-2004/058351
- DE-U1- 20 315 975
- US-A1- 2005 085 799
- US-E1- R E38 533
- HELBIG MEDIZINTECHNIK: "Helbig Katalog 2004/2005" INTERNET CITATION, [Online] 2004, XP002385041 Gefunden im Internet: URL:http://www.helbig.de/Helbig-2004-05-lo w.pdf> [gefunden am 2006-06-12]

## Beschreibung

Die Erfindung betrifft eine Vorrichtung zur Notfallversorgung eines Patienten, die als eine mobile Einheit ausgebildet ist und verschiedene Module mit eigenständiger Funktionalität aufweist, sowie bei der als Module zumindest ein Defibrillator und ein Beatmungsgerät verwendet und auf einer Trageplatte angeordnet sind, die zur Anordnung im Bereich einer Basisstruktur ausgebildet ist.

Bei Notfalleinsätzen ist eine schnelle und adäquate Versorgung des Patienten am Unfallort wichtig. Häufig sind dazu verschiedene medizintechnische Geräte notwendig.

Die verschiedenen medizintechnischen Geräte zur Behandlung/Diagnose des Patienten können sein:
- Defibrillator
- Schrittmacher
- EKG
- Vitalparametermonitor
- Beatmungsgerät
- Pulsoxymeter
- Capnometer/Capnograph
- Respirometer
- Absaugung
- Sauerstoffinhalationsgerät

Von der Fa. Weinmann, Hamburg, sowie anderen Herstellern gibt es bereits Trageinheiten, bei denen eine Sauerstoffquelle und ein Notfallbeatmungsgerät als tragbare Einheit jeweils lösbar auf einer Trageplatte montiert sind.

Die tragbare Einheit bestehend aus Trageplatte und montierten Modulen ist in einem Rettungsfahrzeug, Rettungswagen, Rettungsflugzeug, Rettungshubschrauber, Rettungsboot und anderen Einrichtungen lösbar mit einer Halterung verbunden.

Ein immer wichtiger werdender Defibrillator/Monitor ist meist als Einzelgerät lösbar im Fahrzeug oder im Krankenhaus separat an einen Wand unter-/angebracht.

Die auf der Trageplatte montierten Module verfügen, für die Energieversorgung möglicher elektronischer Funktionen, bevorzugt über einen Akkumulator, welcher über eine Verbindung, Steckkontakt, zwischen der Trageplatte und der Halterung im Rettungsfahrzeug oder dem jeweiligen Anbringungsort geladen werden kann.

Die Beatmung wird zur Fibrillätionsdetektion unterbrochen und nach Schockgabe automatisch wieder aufgenommen, was auch für die Anwendung von Laienhelfer geeignet ist. An die Steuereinheit ist ein Defibrillator und ein Beatmungsgerät angeschlossen, welche beide synchronisiert und auf die Impedanz der angelegten Elektroden angepasst das Beatmungsgerät mindestens teilweise steuert.

Derzeit werden die medizintechnischen Geräte, getrennt voneinander, in mehreren Arbeitsgängen oder mit verschiedenen Tragetaschen zum Patienten gebracht, um diesen am Unfallort zu versorgen. Dies sorgt für einen umständlichen und dadurch langsamen Weg zum Patienten, bei dem in Notfallsituationen wertvolle Zeit verloren geht.

Daneben sind auf Grund vorgeschriebener und/oder empfohlener Wartungsintervalle viele Geräte in Kombination oder als Einzelgerät nicht nutzbar, da die Intervalle verschieden sein können oder verschiedenartig beginnen.

Die vielen Einzelgeräte bedingen auch, sich im Wartungs- und Reparaturfall mit unterschiedlichen Herstellern und Fachhändlern auseinander setzen zu müssen.

Zudem unterliegen die Einzelgeräte vielfach unterschiedlichen Energieversorgungskonzepten, sodass für jedes Einzelgerät eine separate Energieversorgung zum Betrieb des Gerätes bzw. zum Laden seines Akkus angeschlossen werden muss. Je mehr davon vorhanden sind, desto aufwendiger und anfälliger werden bspw. die Bordnetze der Rettungsfahrzeuge; zudem wird die Wartung und Fehlersuche dieser Netze deutlich durch die Vielfalt erschwert.

Daneben gibt es kaum eine Kommunikation zwischen den Geräten, sodass insbesondere die beiden Therapien Beatmung und Defibrillation nur unzureichend abgestimmt werden können. Ein Vorteil der Erfindung ist demnach die Koordination von Beatmungs-, Analyse-, Schock-, Herz-Druck-Massage-Zeiten, welches von einem der Geräte auf der Trageplatte übernommen werden kann. Dies kann durch eine Abstimmung zwischen den gleichberechtigten oder priorisierten Geräten erfolgen.

Zudem ist es für den Anwender sehr schwierig, bei der Vielzahl an verschiedenen Geräten die Bedienkonzepte in Krisensituationen in ausreichendem Maße zu überblicken und zu bedienen.

Bedeutend schwieriger wird es, wenn mindestens zwei Einzelgeräte Alarme ausweisen, sodass der Anwender diese priorisieren muss. Dies setzt zudem einen wachen Patienten unter zusätzlichem Stress und kann seinen Allgemeinzustand deutlich verschlechtern.

Die mangelnde Koordination zwischen Beatmungsgerät und manuell ausgeführter Herzdruckmassage führt zu Druckspitzen in den Atemwegen und löst im Beatmungsgerät vermehrt Alarme aus, was den Anwender zusätzlich unter Druck setzt bzw. eine zusätzliche Lärmquelle verursacht.

Zudem sind die Hands-Off Zeiten bei verschiedenen, nicht kommunizierenden Geräten bisher sehr hoch, d.h. Zeiten, in denen weder beatmet noch eine Herzdruckmassage (HDM) durchgeführt werden kann, da der Defibrillator eine ruhige Analysephase bzw. die Informationen des Beatmungsgerätes und die durch die Beatmung und das Heben und Senken des Brustkorbes verursachten Artefakte und Impedanzänderungen benötigt.

Derzeit geschieht die Datenerfassung bei der Beatmung nur in sehr geringem Maße derart, dass Einstellparameter und besondere Ereignisse nur über eine Papierdokumentation erfaßt werden. Bei Defibrillatoren und Monitoren ist dies bereits üblich.

In der EP-A-0 903 141 wird eine Vorrichtung zur Notfallversorgung eines Patienten beschrieben, die als eine mobile Einheit ausgebildet ist. Die Vorrichtung umfaßt einen Defibrillator und ein Beatmungsgerät und weist eine Trageplatte auf, die im Bereich einer Basisstruktur angeordnet werden kann. Als Basisstruktur kann insbesondere eine rollbare Einrichtung zur Verwendung im Bereich von Kliniken verwendet werden.

In der WO 2004/058351 A wird eine Kombination aus einem Beatmungsgerät und einem Defibrillator beschrieben. Eine ähnliche Anordnung ist auch aus der EP-A-1 264 615 sowie der US-RI 38533 E1 bekannt.

Die Aufteilung spezifischer Funktionen auf eigenständige Module wird beispielsweise in der DE 203 15 975 U1 sowie der Veröffentlichung HELBIG MEDIZINTECHNIK: "Helbig Katalog 2004/2005" INTERNET CITATION, (Online) 2004, XP002385041 gefunden im Internet: URL:http://www.helbig.de/Helbig-2004-05-lo w.pdf> (gefunden am 2006-06-12) beschrieben.

In der EP-A-1 702 649 wird eine weitere Vorrichtung zur Notfallversorgung eines Patienten beschrieben, die verschiedene modulare Funktionseinheiten umfasst. Es werden eine Basisstation zur Anzeige von Daten und mit der Basisstation kommunizierende Module verwendet. Dieses Dokument zeigt jedoch keine Übernahme einer Monitorfunktion durch ein angeschlossenes aktives Gerät mit einer Anzeige sowohl eigener Daten als auch von Daten mindestens eines weiteren Moduls.

Aufgabe der vorliegenden Erfindung ist es, eine Vorrichtung zur Notfallversorgung der einleitend genannten Art derart zu konstruieren, dass eine einfache und zuverlässige Handhabung mit geringem Bedienungsaufwand unterstützt wird.

Diese Aufgabe wird erfindungsgemäß dadurch gelöst, dass ein zentrales Monitoring für Gerätefunktionen und/oder Messdaten implementiert ist, wobei dass mindestens ein Modul sowohl zur Anzeige eigener Daten als auch zur Anzeige von Daten mindestens eines weiteren Moduls ausgebildet ist und bei dem jedes einzelne Modul für seine Funktion zuständig und einzeln funktionsfähig ist, dass jedoch bei weiteren aktiven Geräten eines dieser Geräte die Monitorfunktion, also die Darstellung von Einstellparametern und gemessenen Patienten-Daten und -Werten, übernimmt.

Durch Anordnung der Module im Bereich einer gemeinsamen Trageplatte ist es in einfacher Weise möglich, die Module gemeinsam mit der Trageplatte von der Basisstruktur zu entfernen und einer vorgesehenen Anwendung zuzuführen. Nach dem Abschluss der Anwendung werden die Module gemeinsam mit der Trageplatte wieder in einfacher Weise im Bereich der Basisstruktur befestigt und stehen einer erneuten Anwendung zur Verfügung.

Gemäß der vorliegenden Erfindung kann erstmals die Sauerstoffversorgung, Beatmung und Defibrillation als tragbare Gesamtsystemeinheit für den Notfalleinsatz realisiert werden. Optional sind zusätzlich und oder ergänzend Vitalparametermonitoring und/oder Absaugung vorgesehen. Somit ist Inhalation, Absaugung, Beatmung, einfaches Vitalparametermonitoring und Defibrillation möglich.

Ein weiterer Vorteil der Erfindung ist die Bereitstellung einer kombinierten Dokumentation, insbesondere eine synchronisierte über die Zeit aufgetragene Dokumentation der Therapie.

Die im folgenden aufgezeigten Ausführungsformen sind einzeln oder aber in Kombination anwendbar.

Um die Möglichkeit der modularen Zusammenstellbarkeit auf den Trageplatten sicherzustellen, ist daran gedacht, das MODUL Defibrillation in einer Größe und einem Volumen vergleichbar einem Notfallbeatmungsgeräte wie beispielsweise dem Medumat der Firma WEINMANN, Hamburg zu realisieren.

Für den mobilen Einsatz ist es wichtig, das Gesamtgewicht der Trageplatte + Defibrillator + Beatmung im Bereich von unter 20 kg zu realisieren.

Mit der erfindungsgemäßen Idee wird die Funktionalität von Trageplatten für den Notfalleinsatz um die Defibrillation und/oder ein Vitalparametermonitoring erweitert. Somit ist Inhalation, Absaugung, Beatmung, einfaches Vitalparametermonitoring und Defibrillation möglich. Dies bedeutet für den Anwender eine deutliche Zeitersparnis im Notfalleinsatz, da dieser nicht mehrfach laufen muss, um benötigte Geräte, bspw. aus einem Fortbewegungsmittel, zu holen.

In einer besonders bevorzugte Ausführungsform der Erfindung sind Module wie Defibrillator und Beatmungsgerät mindestens lösbar auf der Trageplatte angebracht, um auch in unwegsamen Gelände, z.B. für die Bergrettung in Schächten die Module transportieren und stellen zu können.

Das Gesamtsystem aus Sauerstoffversorgung, Absaugung, Beatmungsgerät und AED mit Monitoring auf Trageplatte bietet einzigartige Anwendungsvorteile durch Realisierung als tragbare Gesamtsystemeinheit, Beschaffungsvorteile durch flexible, kundenspezifische Zusammenstellbarkeit, jederzeit mögliche Nachrüstbarkeit, Umstellungsvorteile durch eine standardisierte / bereits existierende Fahrzeug- und Ladeschnittstelle (Trageplattensystem) sowie Logistik- und Kostenvorteile durch Service aus einer Hand und durch Vermeidung einer Vielzahl von Wartungsterminen für die Einzelkomponenten.

Erfindungsgemäß ist angedacht, dass die Zusammenstellung der Module sowie die Modulfunktion auf der Trageplatte durch mindestens eines der folgenden Module und/oder Funktionen erweitert werden kann:
- Schrittmacher
- EKG
- Vitalparametermonitor
- Pulsoxymeter
- Pulsspektroskopie
- Capnometer/Capnograph
- Respirometer
- Absaugung
- Sauerstoffinhalationsgerät
- Sauerstoffversorgung
- Monitor
- Energieversorgung über Akkumulatoren

In einer Ausführungsform sind die erfindungsgemäßen Notfalltrageplatten mit abnehmbaren Servicetaschen zur Unterbringung von Verbrauchsmaterialien wie Kathetern, Tuben, Beatmungs- oder Inhalationsmasken angedacht.

Außerdem sind auf den Trageplatten zusätzlich ein Kapnometer oder Pulsoximeter unterbringbar.

Im Rettungsfahrzeug, zum Beispiel an Wand oder Decke, kann zusätzlich eine Halterung/Basisstruktur für die erfindungsgemäßen Notfalltrageplatten eingebaut werden.

Eine Einhand-Entriegelung zur schnellen Entnahme der Notfalltrageplatten aus der Wandhalterung/Basisstruktur dient einfacher Bedienung mit höchster Anwendersicherheit.

Kennzeichnend für die Erfindung ist ein Zentralmonitorkonzept. D.h., dass zwar jedes einzelne Modul für seine Funktion, wie Beatmung, Defibrillation und Analyse, SpO2-Messung, Pulsspektroskopie etc. zuständig und einzeln funktionsfähig ist, dass jedoch bei weiteren aktiven Geräten eines dieser Geräte die Monitorfunktion, also die Darstellung von Einstellparametern und gemessenen Patientendaten und Werten, übernimmt.

In einer weiteren Ausführungsform der Erfindung können über das anzeigende Gerät auch alle anderen aktiven und angezeigten Geräte und Module bedient werden. Die Monitore und Anzeigeeinheiten der aktiven Geräte erlöschen, was die Akku-Laufzeit deutlich erhöht.

In einer Ausführungsform kann der Anwender für jedes Gerät eine Priorität festlegen, so dass er dadurch bestimmen kann, welches Gerät die Monitorfunktion ausführt, inbesondere jeweils das Gerät mit der höchsten Priorität. Voreingestellt ist die Defibrillationseinheit für die Monitorfunktion zuständig. In einer anderen Ausführungsform ist die Beatmungseinheit zuständig für die Monitorfunktion.

In einer besonders bevorzugten Ausführungsform der Erfindung ist angedacht, dass der Monitor als bewegliche, an eines der Geräte ankoppelbare Komponente ausgelegt ist und über eine drahtlose Verbindung verfügt, um Daten mit den Geräten auszutauschen.

Ein weiterer Vorteil der Erfindung ist das gemeinsame Alarmkonzept aller sich auf der Trageplatte befindlichen Geräte.

In einer Ausführungsform der Erfindung sind verschiedene Alarme unterdrückbar und/oder werden durch das Gerät unterdrückt, wenn durch eine Analyseeinheit erkannt wird, dass diese durch gewollte bzw. für den Patienten notwendige Handlungen ausgelöst werden.

In einer anderen Ausführungsform der Erfindung kann beispielsweise der Stenosealarm unterdrückt werden, wenn durch die Kombination der Geräte und Module dem Anwender die Durchführung einer HDM angezeigt wird.

In einer weiteren Ausführungsform kann die Alarmierung über erhöhte Atemwegsdrücke bei einem intubierten Patienten unter Herzdruckmassage unterbunden werden, falls die Impedanzauswertung charakteristisch für die HDM ist.

Eine bevorzugte Ausführungsform der Erfindung erkennt und/oder weist den auftretenden Störungen oder Auslösegründen von Alarmen (Erkannte Unterlassung von HDM, manueller Beatmung, Stenose, erreichter maximaler Beatmungsdruck, etc.) verschiedene Wichtigkeiten zu, welche in den Geräten abgespeichert sein können. So wird in einer Ausführungsform der Erfindung nur der mit der höchsten Wichtigkeit eingestufte Alarm dem Anwender angezeigt. Hat dieser die Ursache des Alarms behoben. So wird, falls vorhanden, der nächst wichtigste Alarm dem Anwender angezeigt.

In einer weiteren Ausführungsform der Erfindung können alle auftretenden Alarme angezeigt werden, jedoch werden diese dann je nach Wichtigkeit geordnet. So hat der Anwender einen Gesamtüberblick über alle Alarme bzw. Probleme, die in der jeweiligen Situation vorherrschen.

Durch diese Alarmmanagements kann eine Anwenderirritation durch zeitgleiche Alarme unterschiedlicher Medizingeräte unterbunden werden.

Erfindungsgemäß ist es also möglich, die Alarme von zwei getrennt voneinander funktionierenden Geräten zu synchronisieren.

Das Zentralmonitorkonzept (Datenübertragung, zentrale Anzeige) ermöglicht die Anzeige aller Überwachungs-parameter, unterschiedlichster Parameter (z.B. et CO2, EKG, SpO2, CO2 und NIBP) sowie die Anzeige von Alarmen und deren Ursprung und/oder Auslösegrund unabhängig von dessen Entstehung (Beatmungsgerät- oder DefiMonitor) an einer gemeinsamen Stelle. Diese Idee erleichtert den Umgang des Anwenders mit einer Fülle von Geräten, da nur noch eine übersichtliche Anzeige alle wichtigen Information bereit stellt.

Erfindungsgemäß weist der Defibrillator und/oder das Beatmungsgerät den Benutzer optisch, beispielsweise über ein Ampelprinzip, und / oder akustisch, beispielsweise über eine Sprachausgabe, auf Funktionszustände und / oder Patientenzustände hin.

In einer weiteren Ausführungsform der Erfindung ist daran gedacht, für die Geräte eine einzige Sprachausgabe bereit zu stellen.

In einer andern Ausführungsform ist daran gedacht, zur Verbesserung der Sicherheit z.B. die Beatmung/Sauerstoffinhalation vor Schockauslösung bei gleichzeitiger Verwendung von Sauerstoff und Defibrillation abzuschalten.

Erfindungsgemäß ist ebenfalls die Anbringung der Trageplatte im Bereich eines Fortbewegungsmittels oder Gebäudes angedacht. Dazu kann die Trageplatte in eine Basisstruktur eingehängt oder anderweitig befestigt werden. Diese Basisstruktur ist fest mit Teilen des Fortbewegungsmittels oder der Gebäude verbindbar.

In einer besonders bevorzugten Form der Erfindung ist die Trageplatte durch eine Ein-Hand-Entriegleung von der Basisstruktur lösbar.

Erfindungsgemäß ist ebenfalls die Anbringung eines Kupplungssystems für eine Ladeschnittstelle und/oder einer Schnittstelle zur Übertragung von Daten angedacht. Dabei verfügen sowohl die Basisstruktur als auch die Tragplatte über Gegenstücke, welche eine Kupplung bzw. eine Schnittstelle für die Durchleitung von Ladestrom bzw. zur Übermittlung von Daten ermöglichen.

Über das Kupplungssystem können die Module während der Haltung durch die Trageplatte in/an der Basisstruktur über die selbe Schnittstelle versorgt und/oder geladen werden.

Die Energieversorgung des Defibrillators und/oder des Beatmungsgerätes auf der Trageplatte erfolgt mit einem oder mehreren Akkumulatoren, der/die über eine Ladeschnittstelle der Trageplatte automatisch aufgeladen wird, sobald sich die Trageplatte in der Basisstruktur im Rettungsfahrzeug befindet.

Gemäß einer weiteren Ausführungsform der Erfindung halten die Trageplatte und die montierten Module Belastungen > 3 g und/oder >10g, bevorzugt jedoch > 20 g, stand. Dies ist auch dann gewährleistet, wenn die Tragplatte an die Basisstruktur angekoppelt ist.

Als großer Vorteil der Erfindung ist die Auswertung der Impedanzinformationen der Klebeelektroden anzusehen, welche auf der Brust des Patienten anbringbar sind. Durch die Auswertung der Impedanz kann somit über den Defibrillator eine Gütebewertung der HDM (Frequenz), die Erkennung von Spontanatmung, die Erkennung des Grades der Eigenatmung und die Erkennung von stattfindender HDM erfolgen.

In einer weiteren Ausführungsform der Erfindung kann der Defibrillator das Beatmungsgerät überwachen; dies kann durch die Impedanzauswertung erfolgen.

Ein weiterer Vorteil der Erfindung ist die Verringerung von Hands-Off-Zeiten. So müssen Defibrillatoren nach dem Stand der Technik zum richtigen Erkennen eines therapiewürdigen bzw. schockwürdigen Herzzustandes an einen komplett ruhigen Patienten angeschlossen sein; die Beatmung ist also während der Analysephase zu unterlassen.

Durch die Kommunikation des Defibrillators mit dem Beatmungsgerät, ermittelt die Analyseeinheit des Defibrillator zu jeder Zeit die Position des Brustkorbes bzw. die aktuelle Beatmungsphase und kann somit auch die charakteristischen Beatmungshubartefakte herausfiltern. Die automatische Arrhythmieerkennung (EKG-Analyse) kann somit auch während der Beatmung, d.h. ohne Beatmungsunterbrechung, durchgeführt werden, so dass der Patient optimal mit Sauerstoff versorgt werden kann. Zudem werden die Impedanzinformationen der angelegten Elektroden zur weiteren Verbesserung der automatischen Arrhythmieerkennung herangezogen.

In einer Ausführungsform der Erfindung ist auch angedacht, dass Beatmungsgerät durch den Defibrillator zu stoppen, um bei schwierigen Analysen eine zuverlässigere artefaktfreie Arrhythmieerkennung durchführen zu können.

In einer weiteren bevorzugten Ausführungsform der Erfindung ist angedacht, HDM-charakteristische Artefakte für eine automatische Arrhythmieerkennung unter Herzdruckmassage (HDM) herauszufiltern.

In einer bevorzugten Ausführungsform der Erfindung ist angedacht, dass die Analysephase direkt nach einer HDM-Phase beginnt. Gleichzeitig können mindestens zwei Beatmungshübe dem Patienten appliziert werden. So kann die Zeit in der keine HDM durchgeführt wird deutlich verringert werden.

So ist angedacht, dass die Zeiten vermieden werden, in denen der Defibrillator nach dem Stand der Technik ohne Kommunikation mit dem Beatmungsgerät eine Analysephase durchführen muss, bei der das Beatmungsgerät keine Beatmung durchführen durfte, da ansonsten die Beatmungshubartefakte die Defibrillation es deutlich erschwerten.

In einer weiteren bevorzugten Ausführungsform gibt der Defibrillator eine Energieladung im Anschluss an die Exspirationsphase, dem Zeitpunkt geringster Impedanz, an den Patienten ab, da Untersuchungen ergeben haben, dass zu diesem Zeitpunkt die besten Ergebnisse für eine Defibrillation erzielbar sind.

Der Defibrillator kann auch durch die Auswertung der Impedanzinformationen den Zeitpunkt des Endes einer Exspiration feststellen. Durch auf den Körper des Patienten angelegte Elektroden wird der Zeitpunkt geringster Impedanz bestimmt und ein Schock/Energieladung abgegeben. Dies kann beatmungsgeräteunabhängig erfolgen.

In einer weiteren bevorzugten Ausführungsform der Erfindung kann die Auswertung der Impedanzmessung über den Defibrillator als Feedbacksystem, z.B. bei der Beutelbeatmung (Frequenz zu hoch/zu niedrig), genutzt werden.

Zudem kann in einer weiteren bevorzugten Ausführungsform der Erfindung die Impedanz über den Defibrillator ausgewertet werden und Aufschluss über optimale Beatmungsparameter (Volumen) geben.

In den Zeichnungen sind Ausführungsbeispiele der Erfindung dargestellt. Es zeigen:
- Fig. 1: Eine Draufsicht auf einen Notfallkoffer mit abgenommenem Verkleidungsdeckel,
- Fig. 2: Eine Darstellung einer Haltevorrichtung der Basisstruktur mit zugeordneter Trageplatte,
- Fig. 3: Eine Darstellung eines Handgriffes der Trageplatte mit zugeordneter Einrastung der Basisstruktur,
- Fig. 4: Eine Darstellung zur Veranschaulichung eines Ergreifens des Handgriffes der Trageplatte und einer Betätigung des Entriegelungsmechanismus mit einem Handgriff,
- Fig. 5: Eine perspektivische Darstellung eines Notfallkoffers,
- Fig. 6: Eine schematische Querschnittdarstellung zur Veranschaulichung der Zuordnung der Trageplatte mit gehalterten Modulen und der Basisstruktur,
- Fig. 7: Eine schematische Darstellung zur Veranschaulichung einer Verschwenkbarkeit der Trageplatte relativ zur Basisstruktur,
- Fig. 8: eine perspektivische Darstellung der Basisstruktur und
- Fig. 9: einen Querschnitt durch die Trageplatte mit befestigter Sauerstofflasche.

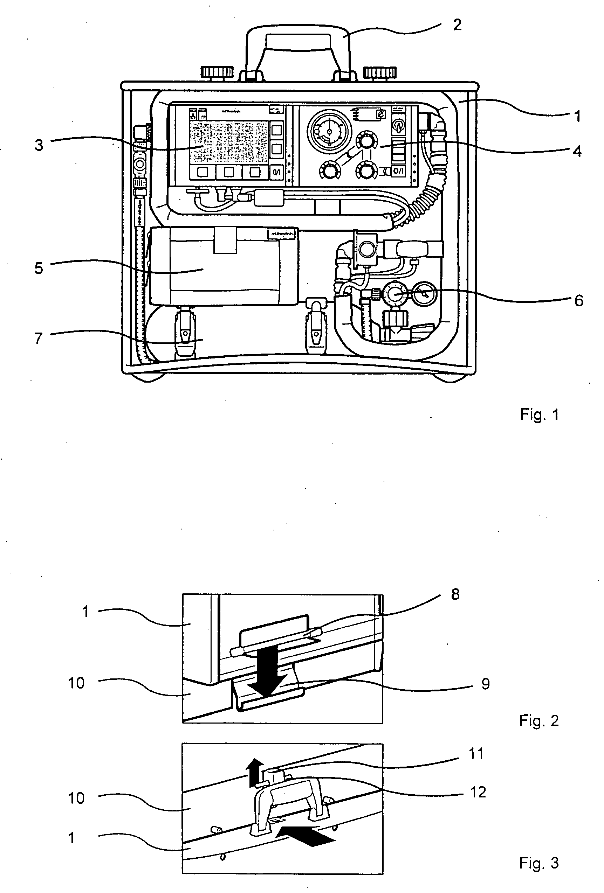

Gemäß der Darstellung in Fig. 1 sind auf einer Trageplatte (1) mit Handgriff (2) ein Defibrillator (3), ein Beatmungsgerät (4), eine Zubehörtasche (5), ein Druckminderer (6) sowie eine Sauerstoffflasche (7) angeordnet. Die Trageplatte (1) weist eine Halterung (8) auf, die in ein Tragelement (9) einer Basisstruktur (10) einhängbar ist. Die Basisstruktur (10) weist darüber hinaus einen Rastmechanismus (11) mit Bedienelement (12) für eine Fixierung der Trageplatte (1) im Bereich der Basisstruktur (10) auf, wie dies in Fig. 3 dargestellt ist.

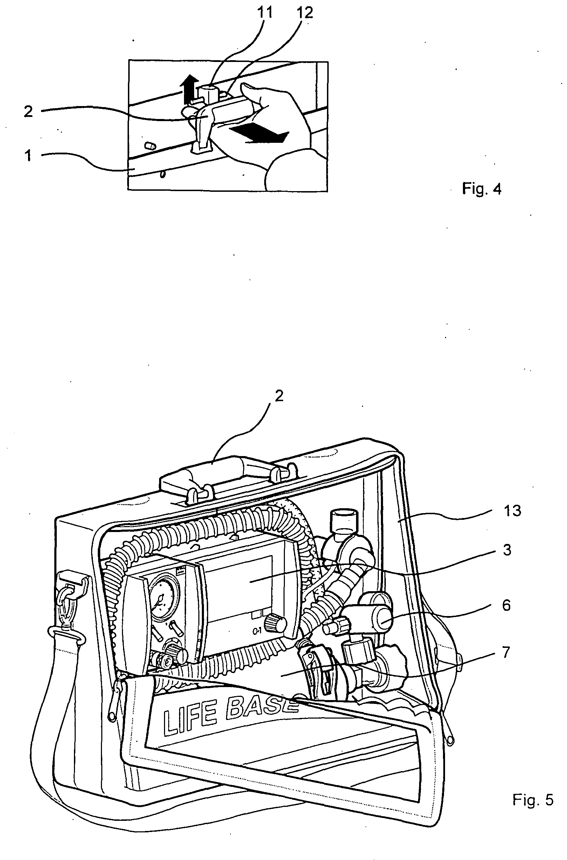

Fig. 4 veranschaulicht eine Betätigung des Rastmechanismus (11) mit Bedienelement (12) beim Ergreifen des Handgriffes (2) der Trageplatte (1).

Fig. 5 zeigt eine perspektivische Anordnung einer Vorrichtung ähnlich der Darstellung in Fig. 1. Die Trageplatte (1) ist in eine kofferartige Umhüllung (13) integriert.

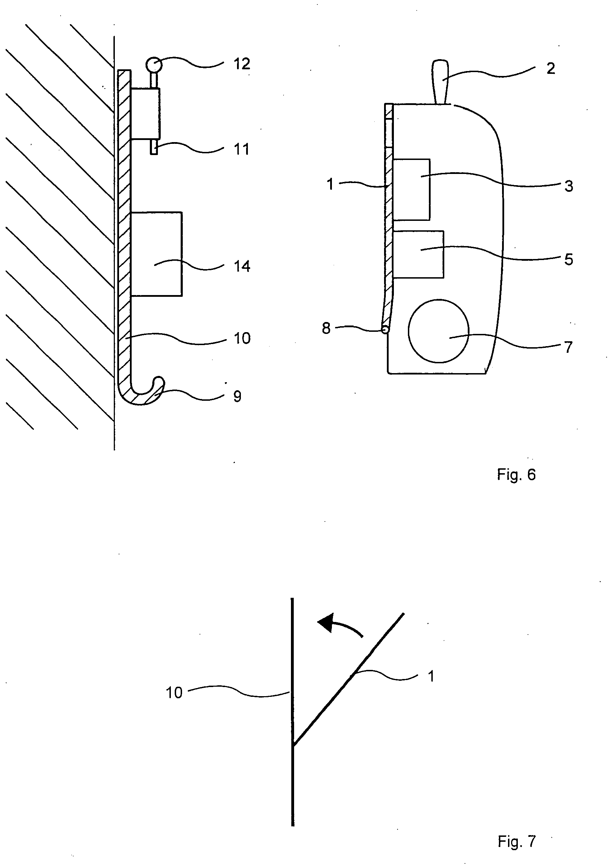

Fig. 6 veranschaulicht noch einmal die Zuordnung der Trageplatte (1) zur Basisstruktur (10). Es ist zu erkennen, daß das Tragelement (9) der Basisstruktur (10) eine hakenartige Anordnung bereitstellt, in die die Trageplatte (1) mit ihrer Halterung (8) eingehängt werden kann. Die Kombination des Tragelementes (9) und der Halterung (8) stellt ein Schwenkgelenk bereit, so daß die Trageplatte anschließend in Richtung auf die Basisstruktur (10) verschwenkt werden kann und im Bereich des Rastmechanismus (11) arretiert wird. Im Bereich der Basisstruktur (10) ist eine Kupplung (14) angeordnet, die zu einer Energieübertragung oder einer Datenübertragung von der Basisstruktur (10) zur Trageplatte (1) bzw. den hier angeordneten Modulen genutzt werden kann.

Fig. 7 veranschaulicht die schwenkbare Anordnung der Trageplatte (1) relativ zur Basisstruktur (10).

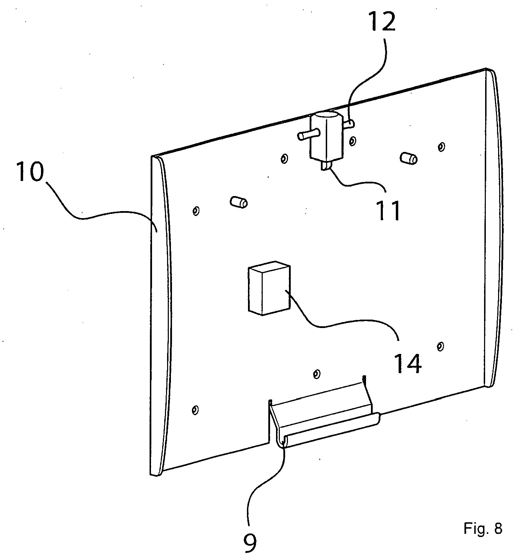

Fig. 8 zeigt eine perspektivische Darstellung der Basisstruktur (10). Zu erkennen sind insbesondere das Tragelement (9) zur Abstützung der Trageplatte (1), der Rastmechanismus (11) mit Bedienelement (12) sowie die Kupplung (14).

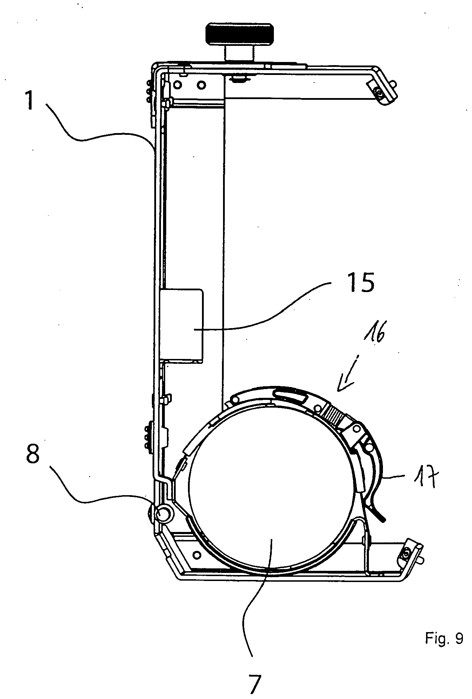

Fig. 9 zeigt die Trageplatte (1) mit Halterung (8) in einer Querschnittdarstellung. Insbesondere ist hier eine Gegenkupplung (15) zu erkennen, die zur Verbindung mit der Kupplung (14) vorgesehen ist. Die Sauerstofflasche (7) ist unter Verwendung eines lösbaren Halteelementes (16) fixiert. Das Halteelement (16) kann beispielsweise als ein Riemen oder Bügel ausgeführt sein, der unter Verwendung eines Handgriffes (17) geöffnet oder verschlossen werden kann. In einem geöffneten Zustand kann die Sauerstofflasche (7) von der Tragplatte (1) getrennt werden.

In einer weiteren Ausführungsform kann das Defibrillationsmodul über die in den folgenden Passagen beschriebenen Funktionen alternativ und/oder ergänzend verfügen:
∘ die Überwachung der Vitalparameter EKG, SpO2, NIBP
∘ die halbautomatische Schockabgabe bei Vorliegen eines Kammerflimmern (VF) oder pulsloser ventrikulärer Tachykardie (VT)
∘ die manuelle Schockabgabe nach Anwenderentscheidung bei Vorliegen einer sonstigen Indikation zur Schockabgabe
∘ die synchronisierte manuelle Schockabgabe (Kardioversion) bei Vorliegen einer supraventrikulären (z.B. Vorhofflimmern) oder ventrikulären Tachykardie.
∘ eine Herzschrittmacherfunktion
∘ biphasische Schockform

Das Defibrillationsmodul funktioniert wie ein herkömmlicher AED mit optischer und sprachgestützter Benutzerführung und EKG-Darstellung. Es ist während des Betriebs umstellbar in einen manuellen Modus.

Die Aufnahme des EKG erfolgt über Defibrillations-Pads und optional über 3-/4-Pol-EKG-Kabel. Dabei können wahlweise entweder nur die Defibrillationselektroden oder das 3-/4-Pol-EKG-Kabel angeschlossen sein, oder aber beide Kabel gleichzeitig. Das EKG wird auf einem Display dargestellt. Im Monitor-Modus werden erkannte QRS-Komplexe als Beep-Ton ausgegeben und die Herzfrequenz dargestellt.

Das Gerät kann über eine Buchse zum Anschluß der SpO2- und Haemoglobin-Sensoren zur Darstellung des Plethysmogramms und der Sättigung und der Pulsfrequenz'verfügen. Außerdem verfügt es über eine drahtlose Kommunikationsschnittstelle zur Übertragung von Daten (z.B. Empfang von NIBP, Versand von Einsatzdaten, Servicedaten).

Das Defibrillationsmodul verfügt über einen Datenspeicher und eine schnelle Übertragungsmöglichkeit für Daten aus dem Speicher, z.B. durch Entnahme einer Speicherkarte oder Funkübertragung. Aufgezeichnet werden Einsatzdaten zur Einsatzdokumentation und -auswertung und zusätzlich Audiodaten.

Für das Defibrillationsmodul ist eine Sprachausgabe vorgesehen. In einem Konfigurationsmodus lässt sich die Landessprache umstellen oder die Sprachausgabe deaktivieren sowie der Reanimationsalgorythmus den jeweils gültigen ILCOR-Empfehlungen anpassen (Schockfolge, HLW-Pausenzeit).

Das Defibrillationsmodul verfügt über einen Schutzgrad von IPX4 gegen ein Eindringen von Wasser.

Die Energieversorgung des Defibrillationsmoduls auf der Trageplatte erfolgt mit einem wechselbaren Akku, der über die Ladeschnittstelle der Trageplatte automatisch aufgeladen wird. Außerdem kann der Akku über ein externes Netzteil geladen werden. Mit dieser Konfiguration ist es dem Anwender möglich, immer einen geladenen Akku als Reserve mitzuführen. Wenn das Defibrillationsmodul einen leeren Akku anzeigt, kann es trotzdem nach Anschluss an die Ladeschnittstelle im Fahrzeug jederzeit Elektroschocks abgeben.

Standzeit: Der Defibrillator ist im Monitoringbetrieb ohne Netzanschluss für zumindest 3 Stunden einsetzbar. Im Schockbetrieb sind ohne Netzanschluss zumindest 40 Schocks und 1,5 Std. Monitoring möglich.

Energieversorgung und Ladung des Defibrillationsmoduls sind mit dem Fahrzeugbordnetz kompatibel (10-18V / 24V).

Es gibt kontextunabhängige Tasten (Hardkeys) und kontextabhängige Tasten (Softkeys). Über die Bedientasten sind folgende Bedienschritte möglich:
- EIN/AUS (Hardkey)
- Menuetaste (Hardkey)
- Moduswahl (AED/manuell/Monitoring/Konfiguration) (Softkey)
- Manueller Start der computerassistierten Arrhythmieerkennung (Softkey)
- HLW-Pause (Softkey)
- Ereignismarkierung (Hardkey/Softkey)
- Energiewahl (Softkey)
- abzugebender Strom im Servicemode einstellbar (Softkey)
- Manuelle Aufladung der Schockenergie (Softkey)
- Schockabgabe (Hardkey)
- Schockenergie verwerfen (Softkey)
- Synchronisierte Defibrillation (Kardioversion, Softkey)
- Ableitungswahl (Softkey)
- Alarmgrenzen einstellen (Softkey)
- Alarm quittieren/stumm schalten (Hardkey)
- Alarm- und Fehlerliste der letzten 10 Ereignisse (technische und Monitoring-Alarme) (Softkey/Hardkey)
- Bedienung des "MODUL Dokumentation" falls verfügbar und auf Trageplatte installiert (Softkey)

Über die Bedientasten können auch folgende Bedienschritte sich ergänzend möglich sein:
∘ EIN/AUS
∘ Moduswahl (AED/manuell/Monitoring/Konfiguration)
∘ Manueller Analysestart
∘ HLW-Pause
∘ Energiewahl
∘ abzugebender Strom im Servicemode einstellbar
∘ Manuelle Aufladung der Schockenergie
∘ Schockabgabe
∘ Schockenergie verwerfen
∘ Synchronisierte Defibrillation (Kardioversion)
∘ Ableitungswahl
∘ Alarmgrenzen einstellen
∘ Alarm quittieren/stumm schalten
∘ Alarm- und Fehlerliste der letzten 10 Ereignisse (technische und Monitoring-Alarme)

Eine Arrythmieerkennung findet in allen Modi statt; es kann ein Arrythmiealarm ausgegeben werden. Im Manuellen- und im Monitoring-Modus erscheint lediglich ein Alarmton als Arrythmiewarnung. Vom Monitoring-Modus aus kann der Anwender sich entscheiden, ob er in den manuellen oder den halbautomatischen (= AED) Defibrillationsmodus wechseln möchte, um das vorliegende Kammerflimmern zu defibrillieren. Im manuellen Modus ist jederzeit eine Energiewahl, manuelle Aufladung und Schockabgabe möglich.

Soll die Rhythmuserkennung computergestützt erfolgen, so kann zur automatischen Rhythmusanalyse in den AED-Modus geschaltet werden. Im AED-Modus kann der Anwender die Analysetaste drücken, um eine computergestützte Rhythmusanalyse zu starten. Dies kann er zu jedem Zeitpunkt im HLW-Ablauf tun. Tut er es nicht, so wird er nach zwei Minuten HLW-Pause eine Aufforderung per Sprachanweisung erhalten, die Analyse-Taste zu drücken.

Ein automatischer Start der computergestützten Rhythmusanalyse nach 2 Min. HLW-Pause erfolgt im AED-Modus nicht. Drückt der Anwender die Taste nicht, wird er im Abstand von z.B. 30 Sekunden oder aber bei erkannter Arrhythmie per Sprachausgabe wiederholt aufgefordert, die Analyse-Taste zu drücken. Der Anwender kann auch jederzeit eine manuelle computergestützte Rhythmusanalyse durch Drücken der Analyse-Taste starten.

Im AED-Easy-Modus erfolgt der computergestützte Analysestart unabhängig von einer vorliegenden Arrhythmie automatisch nach zwei Minuten HLW-Pause oder nach Drücken der Analysetaste.

Das Defibrillationsmodul kann über eine Anzeige/Display verfügen, welches folgende Eigenschaften aufweisen kann:
∘ Farbdisplay mit 320x240 Pixel Auflösung
∘ Bildschirmdiagonale: 5,7 Zoll
∘ Anzeigeverhältnis: 4:3
∘ Größe 116mm x 87mm

Das Display des Defibrillators befindet sich auf der Oberseite des Gehäuses, welches folgende Abmessungen aufweist: Größe von Breite x Länge x Tiefe 125 x 215 x 115 mm. Bevorzugt ist an ein Gehäusevolumen des Defibrillators im Bereich von 3,2 1 oder kleiner, besonders bevorzugt im Bereich von kleiner als 2,8 1 gedacht. In einer weiteren ganz besonders bevorzugten Ausführungsform der Erfindung ist das Gehäusevolumen kleiner als 1,8 1.

Das Gehäuse für den Defibrillator weist bevorzugt im Bereich seiner Rückwand, gegenüberliegend dem Display und den Bedienelementen, Befestigungsvorrichtungen für die lösbare Montage auf einer Trageplatte auf. Die Besfestigungsvorrichtungen sind derart ausgeführt, dass die Befestigung des Defibrillators auf der Trageplatte Stöße und Belastungen bis zu einer Kraft von > 3 g und bevorzugt > 6 g und ganz besonders bevorzugt > 10 g wiederstehen. Das Display und die Bedienelemente des Defibrillators sind durch einen erhöhten Kantenbereich des Gehäuses geschützt. Der Kantenbereich erhebt sich dazu, beispielsweise ausgehend von der Gehäusestruktur, zumindest bereichsweise über die Ebene des Displays und der Bedienelemente.

Bezüglich des Gewichtes des Defibrillationsmodules ist in einer Ausführungsform an ein Gewicht von 5kg, besonders bevorzugt jedoch kleiner 3 kg gedacht.

Die Displaydarstellung kann folgende Informationen bereithalten:
∘ 3 Kurven (EKG-Ableitungen, Phletysmogramm, evtl. weitere)
∘ Herzfrequenz
∘ Pulsfrequenz
∘ Sauerstoffsättigung
∘ Obere und untere Grenzwerte

Das Defibrillationsmodul kann über folgende Schnittestellen eine Anzeige/Display verfügen:
∘ Bluetooth
∘ SD-Karte

Das Beatmungsmodul/Beatmungsgerät weist die in den folgenden Passagen beschriebenen Funktionen alternativ und/oder ergänzend auf: Das Beatmungsmodul/Beatmungsgerät kann neben volumenkontrollierten Beatmungsformen auch druckkontrollierte bzw. -assistierte Beatmungsmodi (CPAP, BiPAP, NIV) ausführen. Damit dient das Beatmungsgerät der Primärversorgung von Notfallpatienten. So ist beispielsweise eine PEEP-Verstellung im Gerät und ohne zusätzliche Zubehörteile angedacht.

Die integrierte PEEP-Steuerung wird mittels Drehknopf auf der Gerätefront bedient. Der PEEP ist in Einstellschritten von 1 hPa zwischen 0 und 15 hPa einstellbar. Das Gerät hat eine Demand Funktion, mit der dem Patienten bei Einatembemühungen ein Sauerstoff-Flow von 8 1/min gegeben wird. Das I:E-Verhältnis ist über Tasten einstellbar.

Das Beatmungsgerät ist zusätzlich mit einer Messung und Anzeige des vom Patienten ein- und ausgeatmeten Atemvolumens ausgestattet.

### Bedienelemente und Frontgestaltung:

### Folgende Parameter sind einstellbar:

- Beatmungsmodus
- Frequenz
- AZV
- Pmax
- Peep
- I:E
- CPAP-Druck
- Hintergrundfrequenz für ASB
- Alarmgrenzen
- Air-Mix on/off
- Alarm-Stumm
- I/O

| | | |
|---|---|---|
| Beatmungsmodus "IPPV" | zeitgesteuert, volumen-konstant, druckbegrenzt Spezielle Form von SW | |
| Beatmungsmodus "SVV" (Smart Volume ventilation) | Intelligente volumenbasierte Beatmung mit zuschaltbarer Triggerung und Druckunterstützung | |
| Beatmungsmodus SPV (Smart Pressure Ventilation) | Intelligente druckbasierte Beatmung mit zuschaltbarer Triggerung und Druckunterstützung | |
| Beatmungsmodus "ASB" | Spontanatmungsunterstüzte Beatmung auf zwei vorgegebenen Beatmungsdrücke | |
| Weitere Beatmungsmodi: | | |
| Volumenkontrolliert: SIMV | | |
| Druckkontrolliert: BiLevel, CPAP, PCV | | |
| Tasten für 3 Schnellstart-Modi | Kleinkind PCV; Kind, Erwachsener IPPV | |
| Inspirations-/Exspirationsverhältnis (I:E) in den 3 IPPV- Modi | 1 : 1,67 oder durch Anwender wählbar | |
| SVV-Einstellparameter: | | |
| Atemzugvolumen (Zielgröße von SW) | 50 - 2000 +/- 15% | ml |
| | 50 - 2000 +/- 10% | ml |
| Atemfrequenz | 0 - 60 | Min⁻¹ |
| I : E | 4:1 bis 1:4 | |
| Bei AF < 6 min⁻¹ Angabe von Tᵢₙₛₚ | | |
| PEEP und EPAP (Pmin) | 0 - 30 | mbar |
| Pₘₐₓ (Sicherheit) | PEEP bis max. 60 | mbar |
| Zuschaltbare Patienten-Triggerung | | |
| Druckunterstützung in der Exsp-60%-Phase (Demand bei 0 mbar) | PEEP + [0..25] | mbar |
| SPV-Einstellparameter: | | |
| Atemfrequenz | 0-60 | min⁻¹ |
| I : E | 4:1 bis 1:3 | |
| EPAP (PEEP) | 0 - 30 | mbar |
| IPAP (Zielgröße von SPV) | PEEP bis max. 60 | mbar |
| AZVₘᵢₙ = Vte_{tief} (Sicherheit) | 5 - 2000 | ml |
| Zuschaltbare Patienten-Triggerung | | |
| Druckunterstützung | P1/P2 + [0..25] | mbar |
| Sonstige Einstellparameter | | |
| Druckanstiegsgeschwindigkeiten für Druckunterstützung | 3 Stufen | |
| Druckanstiegsgeschwindigkeiten für Druckunterstützung | Stufenlos | |
| O₂-Konzentration in 10% Stufen | 40 -100 | % |
| O₂-Konzentration No Air Mix (100%) | 95 -100 | % |
| Air Mix (50%) | 50 - 65 | % |
| Air Mix (50%) | 40 - 50 | % |
| O₂-Konzentration über O₂-Mischer | 21 -100 | % |
| Trigger | | |
| Drucktrigger | | |
| Flowtrigger | | |
| Triggerempfindlichkeit | -0,8 | mbar |
| Triggerempfindlichkeit bei PEEP | unter eingestellten PEEP < 1,5 | mbar |
| Flow - Ansprechverhalten des Triggers | max. 6 | l/mi n |
| Flow - Ansprechverhalten des Triggers | max. 3 | l/mi n |
| Monitoring | | |
| Messung Paw | Patientennah | |
| Atemzugvolumen | Exspiratorische Volumenmessung 20 - 2000 | ml |
| Kapnometrie (Variante) | 20 -75 | mmHg |
| Kapnometrie | 3 -75 | mmHg |
| O₂-Monitoring nach EN 794-1 (Abschn. 51.110) | 15-100 | % FiO₂ |
| Einatemwiderstand während der Spontanatmung (max. 30 l/min simulierter Einatemflow; definiert in EN 794-3) | < 6 | mbar |
| Dito | << 6 (Wert ist zu def.) | mbar |

Die erfindungsgemäße Vorrichtung schließt die Lücke zwischen der reinen Notfallbeatmung und der klinischen Intensivbeatmung in den Bereichen Intermediate Care, Subacute und ICU. Damit ist das Gerät in der Lage, fast alle transportablen differenzierten Beatmungssituationen klinisch wie präklinisch abzudecken und auch in Notfallsituationen einen einfachen Modus anzubieten.

Das Gerät weist neben volumenkontrollierten Beatmungsformen auch druckkontrollierte bzw. -assistierte Beatmungsmodi (CPAP, BiPAP) auf. Damit dient das Beatmungsgerät der Primärversorgung von Notfallpatienten und ist besonders für Sekundärtransporte einsetzbar. In den Beatmungsmodi kann der Anwender für Kleinkinder, Kinder und Erwachsene Voreinstellungen anwählen, um ohne komplizierte Einstellungen das Gerät schnell einsetzen zu können.

Das erfindungsgemäße Beatmungsgerät ist für die Primärversorgung von Notfallpatienten und besonders für die Sekundärversorgung von Transportpatienten an Einsatzorten innerhalb und außerhalb von Gebäuden, zwischen Einsatzort-Klinik und zwischen Kliniken in Ambulanzfahrzeugen, Ambulanzfliegern und Hubschraubern geeignet.

Bei den kontrollierten und auch assistierten Beatmungsmodi sind sensible Trigger und eine Flow-Anpassung/ Steuerung zur Verringerung der Atemwegsspitzendrücke aus atemphysiologischen Gründen realisiert, um dem Anwender die Möglichkeit zu geben, klinische Therapien während des Transportes fortzuführen.

Das Gerät ist für die Primärversorgung von Notfallpatienten wie auch für Sekundärtransporte speziell geeignet. Entsprechend sind folgende Beatmungsmodi realisierbar: IPPV, SIMV nach dem Rechteckflowprinzip und für ASB und BiPAP/CPAP mittels physiologischer Flowkurve. Bei den kontrollierten und auch assistierten Beatmungsmodi ist eine Flow-Anpassung/Steuerung zur Verringerung der Atemwegsspitzendrücke aus atemphysiologischen Gründen notwendig, um dem Anwender die Möglichkeit zu geben, klinische Therapien während des Transportes fortzuführen. Im IPPV-Modus kann der Anwender für Kleinkinder, Kinder und Erwachsene Voreinstellungen anwählen, um ohne komplizierte Einstellungen das Gerät schnell einsetzen zu können. Eine Feineinstellung kann dann zu einem späteren Zeitpunkt vorgenommen werden.

Die Einstellung des I:E-Verhältnisses ist am Gerät möglich. Gleiches gilt für den PEEP. Die Atemwegsdruckbegrenzung wird in zwei Verfahren angeboten: Plateaudruck und Drucklimitierung bei Erreichung des maximalen Begrenzungsdrucks. Das Gerät bietet Monitoring für den Atemwegsdruck, das exspiratorische Atemvolumen und als Variante den exspiratorischen CO2-Wert als Kapnographie.

Akkukapazität und Ladevorgang: Der Akku des GERÄTES muss eine Mindestkapazität von 3 Stunden besitzen. Die Ladespannung des Akkus liegt bei 13,6 .. 16 VDC. Während des Schnell-Ladevorgangs muss das Gerät betrieben werden können.

Datenaufzeichnung und Datentransfer: Die Geräteparameter und die gemessenen Parameter müssen bis zu 8 Stunden aufgezeichnet werden. Eine Trendanzeige reicht aus. Daten werden als einfaches Datenfile (mit Tab getrennt) über USB / Bluetooth ausgegeben.

| | | |
|---|---|---|
| Technische Daten und Toleranzen | | |
| Versorgungsnennspannung (Betrieb bei gleichzeitiger Akkuladung) | 12 und 15 | V DC |
| Toleranzbereich der Versorgungsspannung | 9 bis 16 | V DC |
| Versorgungsnennspannung (evtl. extern) | 100-240 | V AC |
| | 50/60 | Hz |
| Mittlere Strom- bzw. Leistungsaufnahme | Ca. 30 | VA |
| Nennspannung des Akku-Packs | 10,8 | V DC |
| Nennspannung der Batterie (Knopfzelle - Lithium) bzw. Akku oder Goldcap | 3 | V DC |
| Betriebszeit | 3 | H |
| Versorgungsgas im Ausnahmefall | Druckluft | |
| Versorgungsdruck | 2,7 - 6 | Bar |
| Schnittstellen für Datenübertragung | USB, Bluetooth | |
| max. Gewicht | < 5 | Kg |
| Gewicht | < 3 | kg |

Die Größe des Beatmungsmoduls ist kleiner als vorzugsweise Breite x Höhe x Tiefe: 200 x 115 x 120 mm oder kleiner.

Bevorzugt ist an ein Gehäusevolumen von kleiner als 3,2 l, besonders bevorzugt von kleiner als 2,8 l gedacht.

In einer weiteren ganz besonders bevorzugten Ausführungsform der Erfindung ist an ein Gehäusevolumen von kleiner als 1,8 l gedacht.

Bezüglich der Tiefe der Module für die Beatmung und die Defibrillation ist in einer Ausführungsform an eine Tiefe von jeweils 16cm +/- 2 cm oder weniger gedacht, besonders bevorzugt jedoch sind die Module im Bereich 13cm +/- 2 cm tief ausgeführt.

Bezüglich der Breite x Höhe x Tiefe der Basisstruktur ist in einer Ausführungsform eine maximale Breite x Höhe x Tiefe von 600 x 500 x 30 mm angedacht, besonders bevorzugt jedoch 500 x 451 x 25 mm.

Die Abmessungen der Trageplatte sind im Bereich Breite x Tiefe x Höhe 480 x 210 x 340 mm.

Die Trageplatte weist, bevorzugt im Bereich der Grundplatte, Befestigungsvorrichtungen und/oder Aufnahmen für Befestigungsvorrichtungen für die lösbare Montage von zumindest zwei Modulen auf einer Trageplatte auf. Die Befestigungsvorrichtungen sind derart ausgeführt, dass die Befestigung des Defibrillators und des Beatmungsgerätes auf der Trageplatte Stöße und Belastungen bis zu einer Kraft von > 3 g und bevorzugt > 6 g und ganz besonders bevorzugt > 10 g wiederstehen. Das Display und die Bedienelemente des Defibrillators und Display und die Bedienelemente des Beatmungsgerätes sind durch einen erhöhten Kantenbereich der Trageplatte geschützt. Der Kantenbereich erhebt sich dazu, beispielsweise ausgehend von der Gehäusestruktur, zumindest bereichsweise über die Ebene des Displays und der Bedienelemente von Defibrillator und Beatmungsgerät. Bevorzugt ist der Kantenbereich paarig angeordnet und in der Form zweier konvexer Bügel ausgeführt. Der Kantenbereich erhebt sich maximal 200 mm, bevorzugt maximal 170 mm und besonders bevorzugt maximal 140 mm über die Grundfläche der Trageplatte. Dies entspricht einer maximalen Tiefe der Trageplatte von maximal 220 mm, bevorzugt maximal 190 mm und besonders bevorzugt maximal 160 mm. Damit ist die Tiefe der Trageplatte dergestalt, dass sie im Notfalleinsatz bequem einhändig getragen werden kann und im Rettungsfahrzeug, auf der Basisstruktur montiert, nicht zu tief in den Rettungswageninnenraum hinein ragt.

Für den Notfalleinsatz ist es besonders bedeutsam, dass die Einsatzkräfte eine tragbare Einheit aller benötigten Medizingeräte schnell zum Verletzten tragen können und dort die benötigten Medizingeräte in einer kompakten Einheit vorliegen. Dazu müssen die Abmessungen und das Gewicht der benötigten Medizingeräte an die Forderung der Tragbarkeit angepasst sein. Erfindungsgemäß wird daher eine mobile Trageplatte bereitgestellt, auf der zumindest ein Defibrillator und ein Beatmungsgerät montiert sind, wobei die Gesamteinheit der mobilen Trageplatte mit Defibrillator und Beatmungsgerät ein Gewicht von 17 kg nicht übersteigt und bevorzugt ein Gewicht von 13 kg nicht übersteigt und besonders bevorzugt ein Gewicht von 11 kg nicht übersteigt.

Die vorliegende Erfindung ermöglicht es erstmals, eine mobile Trageplatte, auf der zumindest ein Defibrillator und ein Beatmungsgerät montiert sind, als kompakte und tragbare Einheit mit maximalen Abmessungen von im Bereich Breite x Tiefe x Höhe 480 x 210 x 340 mm und einem Gewicht von höchstens 17 kg bereitzustellen.

Bei einer Realisierung der Trageplatte (1) ohne Sauerstofflasche (7) ist es möglich, die Beatmung des Patienten nur unter Verwendung des Beatmungsgerätes und einer Atemmaske durchzuführen. Bei einer zwangsweisen Beatmungsdurchführung kann auch ohne Sauerstoffzugabe eine wirkungsvolle Ventilation des Patienten erreicht werden.

## Patentansprüche

1. Vorrichtung zur Notfallversorgung eines Patienten, die als eine mobile Einheit ausgebildet ist und verschiedene Module mit eigenständiger Funktionalität aufweist, sowie bei der als Module zumindest ein Defibrillator (3) und ein Beatmungsgerät (4) verwendet und auf einer Trageplatte (1) angeordnet sind, die zur Anordnung im Bereich einer Basisstruktur (10) ausgebildet ist, **dadurch gekennzeichnet, dass** ein zentrales Monitoring für Gerätefunktionen und/oder Messdaten implementiert ist, wobei mindestens ein Modul sowohl zur Anzeige eigener Daten als auch zur Anzeige von Daten mindestens eines weiteren Moduls ausgebildet ist und bei dem jedes einzelne Modul für seine Funktion zuständig und einzeln funktionsfähig ist, dass jedoch bei weiteren aktiven Geräten eines dieser Geräte die Monitorfunktion, also die Darstellung von Einstellparametern und gemessenen Patienten-Daten und -Werten, übernimmt.

2. Vorrichtung nach Anspruch 1, **dadurch gekennzeichnet, dass** zusätzlich eine Gasquelle auf der Trageplatte (1) angeordnet ist.

3. Vorrichtung nach Anspruch 2, **dadurch gekennzeichnet, dass** die Gasquelle eine Sauerstoffquelle ist.

4. Vorrichtung nach einem der Ansprüche 2 bis 3, **dadurch gekennzeichnet, dass** die Gasquelle einen zur Umgebung erhöhten Druck aufweist.

5. Vorrichtung nach einem der Ansprüche 1 bis 4, **dadurch gekennzeichnet, dass** für alle Module nur eine Bedieneinheit vorhanden und/oder aktiv ist.

6. Vorrichtung nach einem der Ansprüche 1 bis 5, **dadurch gekennzeichnet, dass** es für alle Module nur ein Alarmkonzept vorhanden ist.

7. Vorrichtung nach einem der Ansprüche 1 bis 6, **dadurch gekennzeichnet, dass** die Basisstruktur (10) im Bereich eines Fortbewegungsmittels oder Gebäudes angebracht ist.

8. Vorrichtung nach einem der Ansprüche 1 bis 7, **dadurch gekennzeichnet, dass** die Basisstruktur (10) und die Trageplatte (1) über ein Kupplungssystem für eine Ladeschnittstelle verfügen, über welche die Module versorgt und/oder geladen werden können.

9. Vorrichtung nach einem der Ansprüche 1 bis 8, **dadurch gekennzeichnet, dass** die Basisstruktur (10) und die Trageplatte (1) über ein Kupplungssystem für eine Kommunikationsschnitt-stelle verfügen, über die beispielsweise die Übertragung von Daten erfolgen kann.

10. Vorrichtung nach einem der Ansprüche 1 bis 9, **dadurch gekennzeichnet, dass** die Zusammenstellung der Module sowie die Modulfunktion durch mindestens eines der folgenden Module und/oder Funktionen erweitert werden kann:
Schrittmacher
EKG
Vitalparametermonitor
Pulsoxymeter
Gasquelle
Pulsspektroskopie
Capnometer/Capnograph
Respirometer
Absaugung
Sauerstoffinhalationsgerät
Sauerstoffversorgung
Monitor
Energieversorgung über Akkumulatoren
Drucker

11. Vorrichtung nach einem der Ansprüche 1 bis 10, **dadurch gekennzeichnet, dass** die Trageplatte (1) eine Ein-Hand-Entriegelung verfügt, um diese von der Basisstruktur (10) abzukoppeln.

12. Vorrichtung nach einem der Ansprüche 1 bis 11, **dadurch gekennzeichnet, dass** alle Module im Zustand der Halterung im Rettungsfahrzeug über die selbe Schnittstelle ladbar sind.

13. Vorrichtung nach einem der Ansprüche 1 bis 12, **dadurch gekennzeichnet, dass** eine zentrale Bedienung für die Funktionsmodule implementiert ist

14. Vorrichtung nach einem der Ansprüche 1 bis 13, **dadurch gekennzeichnet, dass** ein zentrales Alarmmanagement implementiert ist.

15. Vorrichtung nach einem der Ansprüche 1 bis 14, **dadurch gekennzeichnet, dass** mindestens zwei der Module eine gemeinsame Steuerung aufweisen.

16. Vorrichtung nach Anspruch 15, **dadurch gekennzeichnet, dass** die Steuerung mit einer Impedanzauswertung versehen ist.

17. Vorrichtung nach einem der Ansprüche 1 bis 16, **dadurch gekennzeichnet, dass** der Defibrillator (3) in Abhängigkeit von der Impedanzauswertung gesteuert wird.

18. Vorrichtung nach einem der Ansprüche 1 bis 17, **dadurch gekennzeichnet, dass** der Defibrillator (3) in Abhängigkeit von Beatmungsdaten gesteuert wird.

19. Vorrichtung nach einem der Ansprüche 3 bis 18, **dadurch gekennzeichnet, dass** die Sauerstoffquelle als Druckgasflasche ausgebildet ist.

20. Vorrichtung nach einem der Ansprüche 1 bis 19, **dadurch gekennzeichnet, dass** mindestens ein Modul lösbar auf der Trageplatte (1) angeordnet ist.

21. Vorrichtung nach einem der Ansprüche 1 bis 20, **dadurch gekennzeichnet, dass** der Defibrillator (3) mit einer Sprachausgabe versehen ist.

22. Vorrichtung nach einem der Ansprüche 1 bis 21, **dadurch gekennzeichnet, dass** das Beatmungsgerät (4) mit einer Sprachausgabe versehen ist.

23. Vorrichtung nach einem der Ansprüche 1 bis 22, **dadurch gekennzeichnet, dass** der Defibrillator (3) für eine Einsatzfähigkeit in einem Monitoringbetrieb ohne Netzanschluss für zumindest drei Stunden eine interne Energieversorgung aufweist.

24. Vorrichtung nach einem der Ansprüche 1 bis 23, **dadurch gekennzeichnet, dass** der Defibrillator (3) zur Abgabe von mindestens 40 Schocks sowie zur Durchführung eines Monitoring über einen Zeitraum von mindestens 1,5 Stunden ohne Netzanschluss eine Energieversorgung aufweist.

25. Vorrichtung nach einem der Ansprüche 1 bis 24, **dadurch gekennzeichnet, dass** die Energieversorgung und eine Ladeschnittstelle des Defibrillators (3) mit einem Fahrzeugbordnetz kompatibel sind.

26. Vorrichtung nach einem der Ansprüche 1 bis 25, **dadurch gekennzeichnet, dass** ein Gehäusevolumen des Defibrillators (3) maximal 3,2 l beträgt.

27. Vorrichtung nach einem der Ansprüche 1 bis 25, **dadurch gekennzeichnet, dass** ein Gehäusevolumen des Defibrillators (3) maximal 2,8 l beträgt.

28. Vorrichtung nach einem der Ansprüche 1 bis 25, **dadurch gekennzeichnet, dass** ein Gehäusevolumen des Defibrillators (3) maximal 1,8 l beträgt.

29. Vorrichtung nach einem der Ansprüche 1 bis 28, **dadurch gekennzeichnet, dass** der Defibrillator (3) ein Gewicht von höchstens 5 kg aufweist.

30. Vorrichtung nach einem der Ansprüche 1 bis 29, **dadurch gekennzeichnet, dass** der Defibrillator (3) ein Gewicht von höchstens 3 kg aufweist.

31. Vorrichtung nach einem der Ansprüche 1 bis 30, **dadurch gekennzeichnet, dass** ein auf der Trageplatte (1) angeordnete Akkumulator eine Kapazität zur Energieversorgung der Funktionskomponenten von mindestens drei Stunden aufweist.

32. Vorrichtung nach einem der Ansprüche 1 bis 31, **dadurch gekennzeichnet, dass** das Beatmungsgerät (4) und der Defibrillator (3) eine Tiefe von maximal 16 cm ± 2cm aufweisen.

33. Vorrichtung nach einem der Ansprüche 1 bis 32, **dadurch gekennzeichnet, dass** das Beatmungsgerät (4) und der Defibrillator (3) eine Tiefe von maximal 13 cm ± 2cm aufweisen.

34. Vorrichtung nach einem der Ansprüche 1 bis 33, **dadurch gekennzeichnet, dass** die Trageplatte (1) eine Dimensionierung von etwa 480x210x340 mm hinsichtlich Breite x Tiefe x Höhe aufweist.

35. Vorrichtung nach einem der Ansprüche 1 bis 34, **dadurch gekennzeichnet, dass** das Display und die Bedienelemente des Defibrillators (3) sowie die Bedienelemente (12) des Beatmungsgerätes (4) durch einen erhöhten Kantenbereich der Trageplatte (1) umschlossen sind.

36. Vorrichtung nach Anspruch 35, **dadurch gekennzeichnet, dass** sich der Kantenbereich ausgehend von der Gehäusestruktur mindestens bereichsweise über eine von der Oberfläche der umschlossenen Bauteile aufgespannte Ebene erhebt.

37. Vorrichtung nach Anspruch 35 oder 36, **dadurch gekennzeichnet, dass** der Kantenbereich paarig angeordnet und aus zwei konvexen Bügeln ausgebildet ist.

38. Vorrichtung nach einem der Ansprüche 1 bis 37, **dadurch gekennzeichnet, dass** das Gesamtgewicht der Trageplatte (1), des Defibrillators (3) und des Beatmungsgerätes (4) maximal 17 kg beträgt.

39. Vorrichtung nach einem der Ansprüche 1 bis 38, **dadurch gekennzeichnet, dass** das Gesamtgewicht der Trageplatte, des Defibrillators (3) und des Beatmungsgerätes (4) maximal 13 kg beträgt.

40. Vorrichtung nach einem der Ansprüche 1 bis 38, **dadurch gekennzeichnet, dass** das Gesamtgewicht der Trageplatte (1), des Defibrillators (3) und des Beatmungsgerätes (4) maximal 11 kg beträgt.

## Claims

1. Device for emergency care of a patient, which device is designed as a mobile unit and has various modules with independent functionality, and in which device at least a defibrillator (3) and a respirator (4) are used as modules and are arranged on a support plate (1) which is designed for arrangement in the region of a base structure (10), **characterized in that** a central monitoring is implemented for appliance functions and/or measurement data, wherein at least one module is designed both to display its own data and also to display data of at least one further module, and in which each individual module is responsible for its function and is individually functional, **in that** in the case of further active appliances, however, one of these appliances takes over the monitoring function, i.e. the presentation of adjustment parameters and measured patient data and patient values.

2. Device according to Claim 1, **characterized in that** a gas source is additionally arranged on the support plate (1).

3. Device according to Claim 2, **characterized in that** the gas source is an oxygen source.

4. Device according to either of Claims 2 and 3, **characterized in that** the gas source has a pressure higher than the environment.

5. Device according to one of Claims 1 to 4, **characterized in that** only one operating unit is present and/or active for all modules.

6. Device according to one of Claims 1 to 5, **characterized in that** only one alarm concept is present for all modules.

7. Device according to one of Claims 1 to 6, **characterized in that** the base structure (10) is mounted in the region of a means of transport or building.

8. Device according to one of Claims 1 to 7, **characterized in that** the base structure (10) and the support plate (1) have a coupling system for a charging interface via which the modules can be supplied with power and/or charged.

9. Device according to one of Claims 1 to 8, **characterized in that** the base structure (10) and the support plate (1) have a coupling system for a communications interface via which the transmission of data, for example, can take place.

10. Device according to one of Claims 1 to 9, **characterized in that** the combination of the modules and the module function can be extended by at least one of the following modules and/or functions:
pacemaker
ECG
monitor of vital parameters
pulse oximeter
gas source
pulse spectroscopy
capnometer/capnograph
respirometer
aspiration
oxygen inhaler
oxygen supply
monitor
energy supply via accumulators
printer.

11. Device according to one of Claims 1 to 10, **characterized in that** the support plate (1) has a one-hand unlocking mechanism for uncoupling the support plate (1) from the base structure (10).

12. Device according to one of Claims 1 to 11, **characterized in that** all the modules are chargeable via the same interface in the state when mounted in the rescue vehicle.

13. Device according to one of Claims 1 to 12, **characterized in that** a central operation is implemented for the functional modules.

14. Device according to one of Claims 1 to 13, **characterized in that** a central alarm management is implemented.

15. Device according to one of Claims 1 to 14, **characterized in that** at least two of the modules have a common control system.

16. Device according to Claim 15, **characterized in that** the control system is provided with an impedance evaluation.

17. Device according to one of Claims 1 to 16, **characterized in that** the defibrillator (3) is controlled in accordance with the impedance evaluation.

18. Device according to one of Claims 1 to 17, **characterized in that** the defibrillator (3) is controlled in accordance with respiration data.

19. Device according to one of Claims 3 to 18, **characterized in that** the oxygen source is designed as a compressed gas cylinder.

20. Device according to one of Claims 1 to 19, **characterized in that** at least one module is arranged releasably on the support plate (1).

21. Device according to one of Claims 1 to 20, **characterized in that** the defibrillator (3) is provided with a voice output.

22. Device according to one of Claims 1 to 21, **characterized in that** the respirator (4) is provided with a voice output.

23. Device according to one of Claims 1 to 22, **characterized in that** the defibrillator (3) has an internal energy supply to permit use in a monitoring operation for at least three hours without mains connection.

24. Device according to one of Claims 1 to 23, **characterized in that** the defibrillator (3) has an energy supply for outputting at least 40 shocks and for carrying out monitoring over a period of at least 1.5 hours without mains connection.

25. Device according to one of Claims 1 to 24, **characterized in that** the energy supply and a charging interface of the defibrillator (3) are compatible with a vehicle electrical system.

26. Device according to one of Claims 1 to 25, **characterized in that** a housing volume of the defibrillator (3) is at most 3.2 1.

27. Device according to one of Claims 1 to 25, **characterized in that** a housing volume of the defibrillator (3) is at most 2.8 1.

28. Device according to one of Claims 1 to 25, **characterized in that** a housing volume of the defibrillator (3) is at most 1.8 1.

29. Device according to one of Claims 1 to 28, **characterized in that** the defibrillator (3) has a weight of at most 5 kg.

30. Device according to one of Claims 1 to 29, **characterized in that** the defibrillator (3) has a weight of at most 3 kg.

31. Device according to one of Claims 1 to 30, **characterized in that** an accumulator arranged on the support plate (1) has a capacity to supply energy to the functional components for at least three hours.

32. Device according to one of Claims 1 to 31, **characterized in that** the respirator (4) and the defibrillator (3) have a depth of at most 16 cm ± 2 cm.

33. Device according to one of Claims 1 to 32, **characterized in that** the respirator (4) and the defibrillator (3) have a depth of at most 13 cm ± 2 cm.

34. Device according to one of Claims 1 to 33, **characterized in that** the support plate (1) has dimensions of approximately 480 x 210 x 340 mm in terms of width x depth x height.

35. Device according to one of Claims 1 to 34, **characterized in that** the display and the operating elements of the defibrillator (3) and the operating elements (12) of the respirator (4) are enclosed by a raised edge region of the support plate (1).

36. Device according to Claim 35, **characterized in that** the edge region, proceeding from the housing structure, rises at least in sections above a plane spanned by the surface of the enclosed components.

37. Device according to Claim 35 or 36, **characterized in that** the edge region is arranged pairwise and is formed by two convex brackets.

38. Device according to one of Claims 1 to 37, **characterized in that** the overall weight of the support plate (1), defibrillator (3) and respirator (4) is at most 17 kg.

39. Device according to one of Claims 1 to 38, **characterized in that** the overall weight of the support plate, defibrillator (3) and respirator (4) is at most 13 kg.

40. Device according to one of Claims 1 to 38, **characterized in that** the overall weight of the support plate (1), defibrillator (3) and respirator (4) is at most 11 kg.

## Revendications

1. Dispositif destiné à l'alimentation d'urgence d'un patient, qui est réalisé sous la forme d'une unité mobile et qui présente différents modules avec leur fonctionnalité propre, et dans lequel on utilise comme modules au moins un défibrillateur (3) et un appareil respiratoire (4) qui sont installés sur une plaque de support (1), qui est configurée de façon à être disposée dans la région d'une structure de base (10), **caractérisé en ce qu'**une surveillance centrale pour les fonctions des appareils et/ou des données de mesure est mise en oeuvre, dans lequel au moins un module est conçu aussi bien pour l'affichage de ses données propres que pour l'affichage de données d'au moins un autre module, et dans lequel chaque module individuel est responsable de sa fonction et peut fonctionner isolément, **en ce qu'**en cas d'autres appareils actifs un de ces appareils reprend cependant la fonction de surveillance, donc la représentation de paramètres de réglage et de données et de valeurs mesurées du patient.

2. Dispositif selon la revendication 1, **caractérisé en ce qu'**une source de gaz est disposée en outre sur la plaque de support (1).

3. Dispositif selon la revendication 2, **caractérisé en ce que** la source de gaz est une source d'oxygène.

4. Dispositif selon une des revendications 2 à 3, **caractérisé en ce que** la source de gaz présente une pression accrue par rapport à l'environnement.

5. Dispositif selon l'une quelconque des revendications 1 à 4, **caractérisé en ce qu'**il n'y a qu'une seule unité de commande présente et/ou active pour tous les modules.

6. Dispositif selon l'une quelconque des revendications 1 à 5, **caractérisé en ce qu'**il ne se trouve qu'un seul concept d'alarme pour tous les modules.

7. Dispositif selon l'une quelconque des revendications 1 à 6, **caractérisé en ce que** la structure de base (10) est installée dans la région d'un moyen de transport ou d'un bâtiment.

8. Dispositif selon l'une quelconque des revendications 1 à 7, **caractérisé en ce que** la structure de base (10) et la plaque de support (1) sont munies d'un système de couplage pour une interface de charge, par laquelle les modules peuvent être alimentés et/ou chargés.

9. Dispositif selon l'une quelconque des revendications 1 à 8, **caractérisé en ce que** la structure de base (10) et la plaque de support (1) sont munies d'un système de couplage pour une interface de communication, par laquelle on peut effectuer par exemple la transmission de données.

10. Dispositif selon l'une quelconque des revendications 1 à 9, **caractérisé en ce que** la composition des modules ainsi que la fonction des modules peut être étendue par au moins un des modules et/ou fonctions suivants:
stimulateur cardiaque
ECG
moniteur de paramètres vitaux
oxymètre de pouls
source de gaz
spectroscopie d'impulsions
capnomètre/capnographe
respiromètre
aspiration
appareil d'inhalation d'oxygène
alimentation en oxygène
moniteur
alimentation en énergie via des accumulateurs imprimante

11. Dispositif selon l'une quelconque des revendications 1 à 10, **caractérisé en ce que** la plaque de support (1) est munie d'un déverrouillage à une main, afin de détacher celle-ci de la structure de base (10).

12. Dispositif selon l'une quelconque des revendications 1 à 11, **caractérisé en ce que** tous les modules peuvent être chargés via la même interface dans l'état de support dans le véhicule de sauvetage.

13. Dispositif selon l'une quelconque des revendications 1 à 12, **caractérisé en ce qu'**une commande centrale est mise en oeuvre pour les modules fonctionnels.

14. Dispositif selon l'une quelconque des revendications 1 à 13, **caractérisé en ce qu'**une gestion centrale des alarmes est appliquée.

15. Dispositif selon l'une quelconque des revendications 1 à 14, **caractérisé en ce qu'**au moins deux des modules présentent une commande commune.

16. Dispositif selon la revendication 15, **caractérisé en ce que** la commande est munie d'une analyse d'impédance.

17. Dispositif selon l'une quelconque des revendications 1 à 16, **caractérisé en ce que** le défibrillateur (3) est commandé en fonction de l'analyse d'impédance.

18. Dispositif selon l'une quelconque des revendications 1 à 17, **caractérisé en ce que** le défibrillateur (3) est commandé en fonction de données de respiration.

19. Dispositif selon l'une quelconque des revendications 3 à 18, **caractérisé en ce que** la source d'oxygène est réalisée sous la forme d'une bouteille de gaz sous pression.

20. Dispositif selon l'une quelconque des revendications 1 à 19, **caractérisé en ce qu'**au moins un module est disposé de façon détachable sur la plaque de support (1).

21. Dispositif selon l'une quelconque des revendications 1 à 20, **caractérisé en ce que** le défibrillateur (3) est muni d'une sortie vocale.

22. Dispositif selon l'une quelconque des revendications 1 à 21, **caractérisé en ce que** l'appareil respiratoire (4) est muni d'une sortie vocale.

23. Dispositif selon l'une quelconque des revendications 1 à 22, **caractérisé en ce que** le défibrillateur (3) présente une alimentation en énergie interne pour une utilisation en fonctionnement de surveillance sans raccordement au réseau pendant au moins trois heures.

24. Dispositif selon l'une quelconque des revendications 1 à 23, **caractérisé en ce que** le défibrillateur (3) présente une alimentation en énergie pour la production d'au moins 40 chocs ainsi que pour l'exécution d'une surveillance pendant une période de temps d'au moins 1,5 heures sans raccordement au réseau.

25. Dispositif selon l'une quelconque des revendications 1 à 24, **caractérisé en ce que** l'alimentation en énergie et une interface de charge du défibrillateur (3) sont compatibles avec un réseau de bord d'un véhicule.

26. Dispositif selon l'une quelconque des revendications 1 à 25, **caractérisé en ce qu'**un volume de boîtier du défibrillateur (3) vaut au maximum 3,2 1.

27. Dispositif selon l'une quelconque des revendications 1 à 25, **caractérisé en ce qu'**un volume de boîtier du défibrillateur (3) vaut au maximum 2,8 1.

28. Dispositif selon l'une quelconque des revendications 1 à 25, **caractérisé en ce qu'**un volume de boîtier du défibrillateur (3) vaut au maximum 1,8 1.

29. Dispositif selon l'une quelconque des revendications 1 à 28, **caractérisé en ce que** le défibrillateur (3) présente un poids maximum de 5 kg.

30. Dispositif selon l'une quelconque des revendications 1 à 29, **caractérisé en ce que** le défibrillateur (3) présente un poids maximum de 3 kg.

31. Dispositif selon l'une quelconque des revendications 1 à 30, **caractérisé en ce qu'**un accumulateur disposé sur la plaque de support (1) présente une capacité d'alimentation en énergie des composants fonctionnels d'au moins trois heures.

32. Dispositif selon l'une quelconque des revendications 1 à 31, **caractérisé en ce que** l'appareil respiratoire (4) et le défibrillateur (3) présentent une profondeur maximale de 16 cm ± 2 cm.

33. Dispositif selon l'une quelconque des revendications 1 à 32, **caractérisé en ce que** l'appareil respiratoire (4) et le défibrillateur (3) présentent une profondeur maximale de 13 cm ± 2 cm.

34. Dispositif selon l'une quelconque des revendications 1 à 33, **caractérisé en ce que** la plaque de support (1) présente des dimensions d'environ 480x210x340 mm concernant largeur x profondeur x hauteur.

35. Dispositif selon l'une quelconque des revendications 1 à 34, **caractérisé en ce que** l'écran d'affichage et les éléments de commande du défibrillateur (3) ainsi que les éléments de commande (12) de l'appareil respiratoire (4) sont entourés par une région relevée des côtés de la plaque de fond (1).

36. Dispositif selon la revendication 35, **caractérisé en ce que** la région des côtés partant de la structure du boîtier s'élève au moins localement au-dessus d'un plan défini par la face supérieure des composants entourés.

37. Dispositif selon la revendication 35 ou 36, **caractérisé en ce que** la région des côtés est disposée par paires et est formée de deux étriers convexes.

38. Dispositif selon l'une quelconque des revendications 1 à 37, **caractérisé en ce que** le poids total de la plaque de support (1), du défibrillateur (3) et de l'appareil respiratoire (4) vaut au maximum 17 kg.

39. Dispositif selon l'une quelconque des revendications 1 à 38, **caractérisé en ce que** le poids total de la plaque de support, du défibrillateur (3) et de l'appareil respiratoire (4) vaut au maximum 13 kg.

40. Dispositif selon l'une quelconque des revendications 1 à 38, **caractérisé en ce que** le poids total de la plaque de support (1), du défibrillateur (3) et de l'appareil respiratoire (4) vaut au maximum 11 kg.
